# FASCICULE DE BREVET EUROPEEN

(11) **EP 3 573 549 B1**
(45) Date de publication et mention de la délivrance du brevet: **21.02.2024**
(21) Numéro de dépôt: 18705687.4
(22) Date de dépôt: 29.01.2018
(51) Int. Cl.: A61B 17/32, A61C 1/07, A61B 17/00

(54) **APPAREIL DE TRAITEMENT À ULTRASONS AVEC CONTRÔLE AUTOMATIQUE DE CONSIGNE**
ULTRASCHALLBEHANDLUNGSVORRICHTUNG MIT AUTOMATISCHER SOLLWERTSTEUERUNG
ULTRASOUND TREATMENT APPARATUS WITH AUTOMATIC SETPOINT CONTROL

(30) Priorité: 30.01.2017 FR 1750730
(43) Date de publication de la demande: 04.12.2019
(73) Titulaire: Société pour la Conception des Applications des Techniques Electroniques - SATELEC, 33700 Mérignac (FR)
(72) Inventeur: CAPET, Xavier, 33610 Cestas (FR); RECHE, Charles, 33000 Bordeaux (FR)
(74) Mandataire: Cabinet Beau de Loménie
(86) Numéro de dépôt international: PCT/FR2018/050198
(87) Numéro de publication internationale: WO 2018/138453

(56) Documents cités:
- EP-A1- 2 138 123
- US-A1- 2010 231 090
- US-A1- 2013 282 038
- US-A1- 2016 120 563
- US-A1- 2016 325 121
- Satelec: "Piezotome Solo", , 31 décembre 2011 (2011-12-31), XP055423942, Extrait de l'Internet: URL:http://www.chirurgie-dentaire-osseuse. com/documentation/doc-commerciales/PZT Solo_D57500_FR.pdf [extrait le 2017-11-10]
- Acteon: "Livret clinique Chirurgie", , 31 décembre 2012 (2012-12-31), XP055423947, Extrait de l'Internet: URL:http://www.chirurgie-dentaire-osseuse. com/documentation/doc-cliniques/Livret_cli nique_FR.pdf [extrait le 2017-11-10]

## Description

### Arrière-plan de l'invention

La présente invention concerne les appareils de traitement à ultrasons utilisés notamment dans le domaine dentaire tels que les appareils de détartrage, de surfaçage (élimination de biofilms) ou de taille (cavités ou préparations de prothèse), ou dans le domaine chirurgical (par exemple : chirurgie maxillo-faciale et stomatologie ou chirurgie orthopédique). Ces appareils comprennent des instruments vibrant à des fréquences ultrasonores.

Les appareils de traitement à ultrasons, comme ceux divulgués notamment dans les documents US 2013/282038 et EP 2 138 123, comprennent généralement une pièce à main chirurgicale, un insert ou outil ultrasonique et un générateur d'ultrasons. La pièce à main comprend un transducteur piézoélectrique constitué d'une partie distale sur laquelle est fixé l'insert ultrasonique et d'un moteur piézoélectrique relié au générateur d'ultrasons et couplé mécaniquement à la partie distale. Le moteur piézoélectrique transmet à l'insert des ondes ultrasonores définies en fonction de signaux de consigne de courant et de tension délivrés par le générateur d'ultrasons au moteur piézoélectrique. Les signaux de consigne sont fixés en début de traitement, leur valeur étant déterminée en fonction du type traitement à réaliser. Par exemple, dans le domaine dentaire, pour un débridement parodontal, la consigne en courant et tension est nettement inférieure à celle nécessaire pour un détartrage. Par conséquent, pour chaque type de traitement dentaire, il existe une consigne adaptée pour le contrôle des ondes ultrasonores.

Cependant, lors d'un traitement, l'insert ultrasonique peut rencontrer des milieux ou matériaux ayant des duretés différentes. L'insert peut par exemple rencontrer à la fois des tissus mous comme une gencive, un muscle, etc. et des tissus plus durs comme un os. La valeur de la consigne est la même pour un type de traitement, et ce quel que soit les milieux ou matériaux rencontrés par l'insert durant le traitement.

Par conséquent, au cours d'un même traitement, il se peut que la consigne fixant l'amplitude des signaux de consigne de courant et de tension délivrés par le générateur d'ultrasons ne soit pas adaptée ou optimale par rapport au milieu ou matériau rencontré par l'insert, ce qui peut conduire, par exemple, à des risques d'endommagements des parties fragiles en contact avec l'insert ou à un manque d'efficacité du traitement lorsque l'insert est en contact avec des matériaux très résistants.

### Objet et résumé de l'invention

La présente invention a pour but de remédier aux inconvénients précités et de proposer une solution qui permet d'adapter les signaux de consigne délivrés au transducteur à la dureté du matériau en contact avec l'insert ultrasonique.

Ce but est atteint grâce à un appareil de traitement à ultrasons comprenant au moins une pièce à main chirurgicale, un insert ultrasonique et un générateur d'ultrasons, la pièce à main comprenant un transducteur piézoélectrique constitué d'une partie distale sur laquelle est fixé l'insert ultrasonique et d'un moteur piézoélectrique relié au générateur d'ultrasons et couplé mécaniquement à la partie distale, ledit moteur piézoélectrique transmettant à l'insert des ondes ultrasonores définies en fonction de signaux de consigne de courant et de tension délivrés par le générateur d'ultrasons au moteur piézoélectrique, ledit appareil de traitement à ultrasons comprenant en outre un module de contrôle d'amplitude des signaux de consigne configuré pour :
- calculer une variation de la fréquence et de l'impédance d'un signal ultrasonore dans le transducteur piézoélectrique,
- comparer la variation de l'impédance du signal ultrasonore à une valeur prédéterminée de variation d'impédance,
- comparer la variation de la fréquence du signal ultrasonore à une valeur prédéterminée de variation de fréquence,
- augmenter l'amplitude des signaux de consigne de courant et de tension délivrés par le générateur d'ultrasons lorsque la variation de l'impédance du signal ultrasonore calculée est supérieure à la valeur prédéterminée de variation d'impédance et lorsque la variation de la fréquence du signal ultrasonore calculée est supérieure à la valeur prédéterminée de variation de fréquence, et
- diminuer l'amplitude des signaux de consigne de courant et de tension délivrés par le générateur d'ultrasons lorsque la variation de l'impédance du signal ultrasonore calculée est supérieure à la valeur prédéterminée de variation d'impédance et lorsque la variation de la fréquence du signal ultrasonore calculée est inférieure à la valeur prédéterminée de variation de fréquence.

Ainsi, l'appareil de traitement dentaire selon l'invention est capable de détecter le changement de dureté du matériau en contact avec l'insert ultrasonique et de modifier automatiquement l'amplitude des signaux de consigne délivrés au transducteur en fonction de la dureté du matériau. L'appareil de l'invention assiste automatiquement le geste du praticien qui bénéficie ainsi d'une sécurité améliorée et d'une plus grande efficacité dans son travail.

La présente invention est en outre remarquable en ce que le changement de dureté est détecté sans avoir à équiper la pièce à main de capteurs supplémentaires. En effet, le changement de dureté est détecté en fonction des variations d'impédance et de fréquence dans le transducteur, les variations étant dépendantes du matériau en contact avec l'insert ultrasonique. Ce dernier joue le rôle de capteur, ce qui permet une précision de mesure optimale.

La variation de la fréquence et de l'impédance du signal ultrasonore dans le transducteur piézoélectrique comprend la mesure d'au moins deux valeurs moyennes de la fréquence et de l'impédance du signal ultrasonore dans le transducteur piézoélectrique sur une période déterminée, la variation de la fréquence du signal ultrasonore étant calculée entre deux valeurs moyennes mesurées de la fréquence du signal ultrasonore, la variation de l'impédance du signal ultrasonore étant calculée entre deux valeurs moyennes mesurées de l'impédance du signal ultrasonore. Les valeurs moyennes de la fréquence et de l'impédance du signal ultrasonore dans le transducteur piézoélectrique peuvent être mesurées toutes les 100 millisecondes. Dans ce cas, selon un aspect du procédé, la valeur prédéterminée de variation de fréquence peut être égale à 12 hertz et la valeur prédéterminée de variation d'impédance peut être égale à 26 ohms.

Selon encore un autre aspect de l'appareil de l'invention, l'amplitude des signaux de consigne de courant et de tension délivrés par le générateur d'ultrasons est augmentée de 20% lorsque la variation de la fréquence du signal ultrasonore calculée est supérieure à la valeur prédéterminée de variation de fréquence et lorsque la variation de l'impédance du signal ultrasonore calculée est supérieure à la valeur prédéterminée de variation d'impédance. De même, l'amplitude des signaux de consigne de courant et de tension délivrés par le générateur d'ultrasons est diminuée de 20% lorsque la variation de la fréquence du signal ultrasonore calculée est inférieure à la valeur prédéterminée de variation de fréquence et lorsque la variation de l'impédance du signal ultrasonore calculée est supérieure à la valeur prédéterminée de variation d'impédance.

### Brève description des dessins

D'autres caractéristiques et avantages de l'invention ressortiront de la description suivante de modes particuliers de réalisation de l'invention, donnés à titre d'exemples non limitatifs, en référence aux dessins annexés, sur lesquels :
- la figure 1 est une vue schématique d'un appareil de traitement dentaire à ultrasons,
- la figure 2 est une vue schématique en coupe de la pièce à main de l'appareil de la figure 1,
- la figure 3 est un schéma fonctionnel d'un circuit électronique de commande du générateur d'ultrasons de l'appareil de la figure 1 conformément à un mode de réalisation de l'invention,
- la figure 4 est un ordinogramme montrant des étapes d'un procédé de contrôle des signaux de consigne dans un appareil de traitement à ultrasons
- la figure 5 est un graphique montrant les conditions du contrôle de l'amplitude des signaux de consigne,
- la figure 6 est un exemple de mise en oeuvre du contrôle d'amplitude des signaux de consigne.

### Description détaillée des modes de réalisation de l'invention

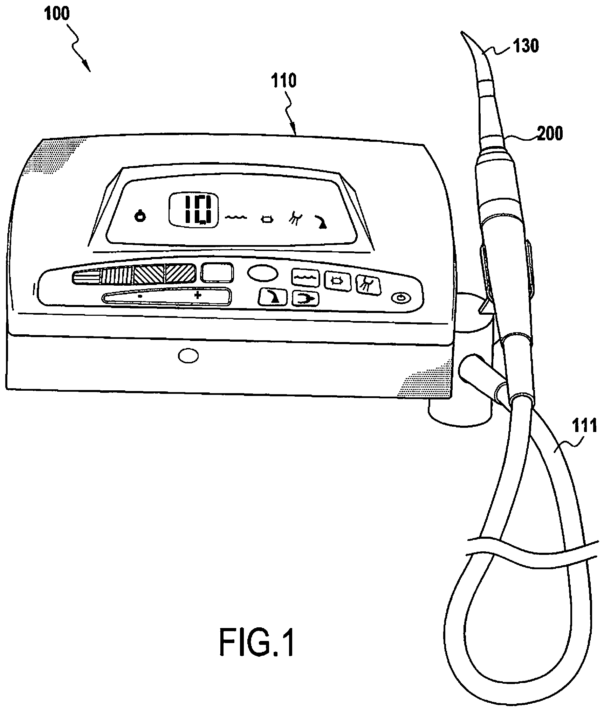

L'appareil de traitement à ultrasons de l'invention trouve des applications dans le domaine générale de la chirurgie et plus particulièrement dans le domaine de la chirurgie ou des traitements dentaires ainsi que de la chirurgie osseuse (exemple : chirurgie maxillo-faciale ou orthopédique). La figure 1 illustre un appareil de traitement à ultrasons 100 qui comprend un générateur d'ultrasons 110 relié à une pièce à main 200 par un cordon 111. Une sonotrode ou insert ultrasonique 130 est monté sur la partie supérieure ou distale de la pièce à main 200. De façon bien connue, la pièce à main 200 comprend un transducteur (non représenté sur la figure 1) formé d'un matériau piézoélectrique et couplé mécaniquement à l'insert 130 de manière à transmettre à ce dernier des ondes ultrasonores définies en fonction de signaux de consigne de courant et de tension délivrés par le générateur d'ultrasons.

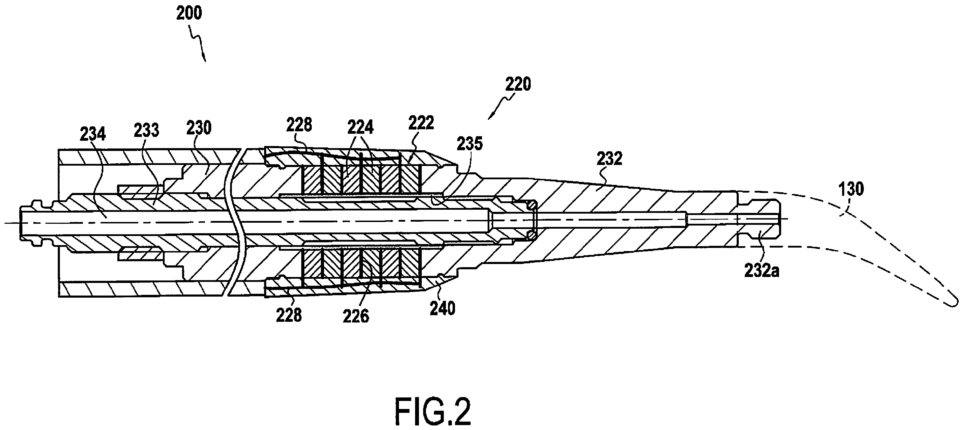

La figure 2 est une vue en coupe longitudinale représentant la structure interne du transducteur piézoélectrique 220 disposé dans la pièce à main 200. Ce transducteur piézoélectrique comprend notamment une partie proximale 230, une partie distale 232 et un moteur piézoélectrique 222 intercalé entre les parties distale et proximale. Dans l'exemple considéré ici, les parties proximale 230 et distale 232 correspondent respectivement à une contremasse et à un amplificateur. La contremasse 230 et l'amplificateur 232 sont en appui mécanique contre le moteur piézoélectrique 222 de manière ce que ce dernier soit contraint mécaniquement.

Dans cet exemple, le moteur piézoélectrique 222 est constitué de 6 céramiques 224 piézoélectriques de forme annulaire disposées les unes contre les autres sous contrainte entre la contremasse 230 et l'amplificateur 232. Chacune des céramiques 224 est recouverte d'un revêtement électriquement conducteur (en argenture, par exemple) et 7 plaques annulaires conductrices 226 sont intercalées entre les céramiques 224 de manière à relier électriquement les câbles d'alimentation 228 avec les céramiques 224 piézoélectriques. Lorsque les céramiques 224 du moteur piézoélectrique 222 sont soumises à un signal électrique transmis par les câbles 228 reliés au générateur d'ultrasons, elles génèrent des vibrations mécaniques sous forme d'ondes ultrasonores. Ces ondes ultrasonores sont alors transmises via la partie distale 232 du transducteur piézoélectrique 220 vers l'insert ultrasonique 130 (non représenté sur la figure 2) monté à l'extrémité libre 232a de la partie distale 232.

Dans cet exemple, un enrobage électriquement isolant 240 est par ailleurs disposé autour du moteur piézoélectrique 222 afin notamment de protéger et d'isoler les câbles 228. Par ailleurs, une tige rigide précontrainte 233 est disposée au centre du transducteur piézoélectrique 220. Un élément 235 électriquement isolant est en outre disposé entre la tige précontrainte 233 et le moteur piézoélectrique 222. Un conduit d'irrigation 234 est en outre formé au centre de la tige précontrainte 233 de manière à pouvoir faire circuler un liquide dans la pièce à main et le délivrer en sortie de l'insert 130.

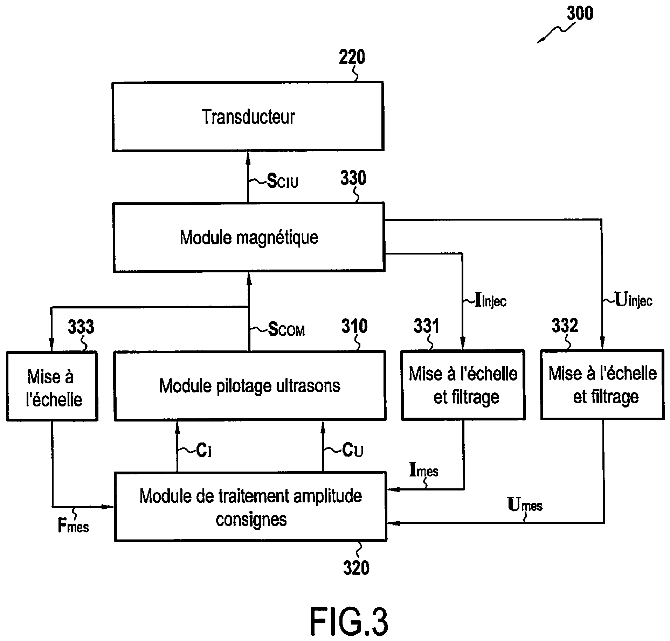

Conformément à l'invention, l'appareil de traitement à ultrasons est en outre apte à mesurer la variation d'impédance et d'amplitude du signal ultrasonore dans le transducteur piézoélectrique et de modifier les signaux de consigne délivrés par le générateur d'ultrasons lorsque la variation d'impédance du signal ultrasonore dépasse une valeur prédéterminée de variation d'impédance et lorsque la variation de fréquence du signal ultrasonore passe en dessous ou au-dessus d'une valeur prédéterminée de variation d'impédance. A cet effet, l'appareil de traitement comprend un module de traitement d'amplitude des signaux de consigne configuré pour réaliser les opérations mentionnées ci-avant. La figure 3 est un schéma fonctionnel d'un circuit électronique de commande 300 du générateur d'ultrasons équipé d'un module de traitement d'amplitude des signaux de consigne conformément à un mode de réalisation de l'invention. Le circuit de commande 300 comprend un module de pilotage d'ultrasons 310 qui est configuré pour envoyer au transducteur 220 de la pièce à main via les câbles 228 des signaux de consigne en courant et en tension S_{CIU} qui définissent la fréquence et l'amplitude des ondes ultrasonores produites par le moteur piézoélectrique du transducteur, la vitesse de vibration du moteur piézoélectrique étant une fonction directe du courant électrique qui circule dans celui-ci tandis que l'effort nécessaire à cette vibration est une fonction directe de la tension d'alimentation. Les signaux de consigne Sau sont définis au départ en fonction du type traitement à réaliser. Les signaux de consigne en courant et en tension S_{CIU} sont transmis au transducteur via un module magnétique 330 qui comprend un transformateur (non représenté sur la figure 3). Les signaux de consignes S_{COM} délivrés par module de pilotage d'ultrasons 310 sont transformés par le module magnétique en signaux de consigne en courant et en tension S_{CIU}. Conformément à l'invention, le circuit électronique de commande 300 comprend en outre un module de contrôle d'amplitude des signaux de consigne 320 qui mesure la variation de la fréquence et de l'impédance du signal ultrasonore dans le transducteur piézoélectrique. A cet effet, le module 320 reçoit des signaux Iₘₑₛ et Uₘₑₛ correspondant respectivement au courant et à la tension dans le transducteur. L'image du courant et de la tension dans le transducteur 220 est obtenue en mesurant la valeur moyenne du courant I_{injec} et de la tension U_{injec} injectés dans le transducteur. Les signaux injectés I_{injec} et U_{injec} sont prélevés sur le primaire du module magnétique 330. Après mise à l'échelle et filtrage de ces signaux par respectivement par des modules 331 et 332, on obtient les signaux de mesure du courant et de tension dans le transducteur Iₘₑₛ et Uₘₑₛ. La mesure du courant et de la tension dans le transducteur peut ainsi être réalisée sans avoir à se connecter au borne du transducteur, c'est-à-dire sans câblage supplémentaire dans l'appareil à ultrasons. La fréquence et l'impédance du signal ultrasonore dans le transducteur varie en fonction de la dureté du matériau en contact avec l'insert ultrasonique. L'image de la fréquence dans le transducteur 220 est obtenue à partir des signaux de consignes S_{COM} délivrés par module de pilotage d'ultrasons 310. Après mise à l'échelle par un module 333, on obtient un signal de mesure de la fréquence dans le transducteur Fₘₑₛ.

Le module 320 calcule l'impédance dans le transducteur à partir des signaux Iₘₑₛ et Uₘₑₛ. Le module 320 reçoit également le signal Fₘₑₛ correspondant à la fréquence dans le transducteur. Le module 320 peut ainsi mesurer en permanence l'impédance et la fréquence du signal dans le transducteur et en déduire une valeur moyenne sur une période déterminée, par exemple toutes les 100 millisecondes. Le module 320 calcule la variation de l'impédance et de la fréquence à partir de deux mesures consécutives.

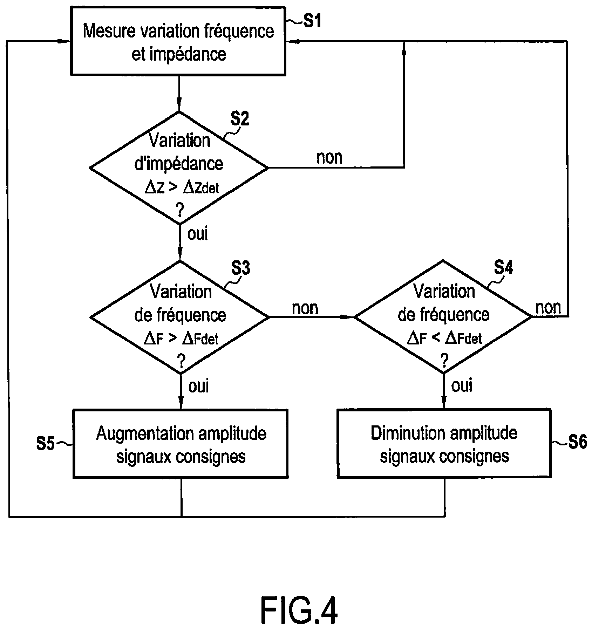

On décrit maintenant en relation avec la figure 4 les étapes du contrôle des consignes par le module de contrôle d'amplitude des signaux de consigne 320. Comme indiqué ci-avant, le module 320 détermine une valeur moyenne de mesure de l'impédance et la fréquence du signal dans le transducteur sur une période déterminée et calcule leur variation entre deux valeurs moyennes de mesures consécutives (étape S1). Le module 320 compare la variation de l'impédance Δz calculée à une valeur prédéterminée de variation d'impédance Δ_{Zdet} (étape S2). Si la variation de l'impédance calculée Δ_{Z} est inférieure à la valeur prédéterminée de variation d'impédance Δ_{Zdet}, le module 320 laisse les signaux de consigne inchangés. Si la variation de l'impédance calculée Δ_{Z} est supérieure à la valeur prédéterminée de variation d'impédance Δ_{Zdet}, le module 320 compare la variation de fréquence calculée Δ_{F} à une valeur prédéterminée de variation de fréquence Δ_{Fdet} (étapes S3 et S4). Plus précisément, le module 320 détermine si la variation de fréquence calculée Δ_{F} est supérieure à la valeur prédéterminée de variation de fréquence Δ_{Fdet} (étape S3). Si c'est le cas, le module 320 envoie des signaux de contrôle C_{I} et Cu au module de pilotage des signaux de consigne 310 pour que celui-ci augmente l'amplitude du signal de consigne en courant et en tension S_{CIU} délivré transducteur 220 (étape S5). Dans le cas contraire, le module 320 détermine si la variation de fréquence calculée Δ_{F} est inférieure à la valeur prédéterminée de variation de fréquence Δ_{Fdet} (étape S4). Si c'est le cas, le module 320 envoie des signaux de contrôle C_{I} et Cu au module de pilotage des signaux de consigne 310 pour que celui-ci diminue l'amplitude des signaux de consigne en courant et en tension S_{CIU} délivrés au transducteur 220 (étape S6). Dans le cas contraire, le module 320 laisse les signaux de consigne inchangés. Les étapes S3 et S4 peuvent être réalisées dans un ordre quelconque.

L'augmentation et la diminution de l'amplitude des signaux de consigne sont commandées pour un temps de commande déterminé, par exemple 415 millisecondes. Une fois le temps de commande déterminé dépassé, on revient à l'étape S1.

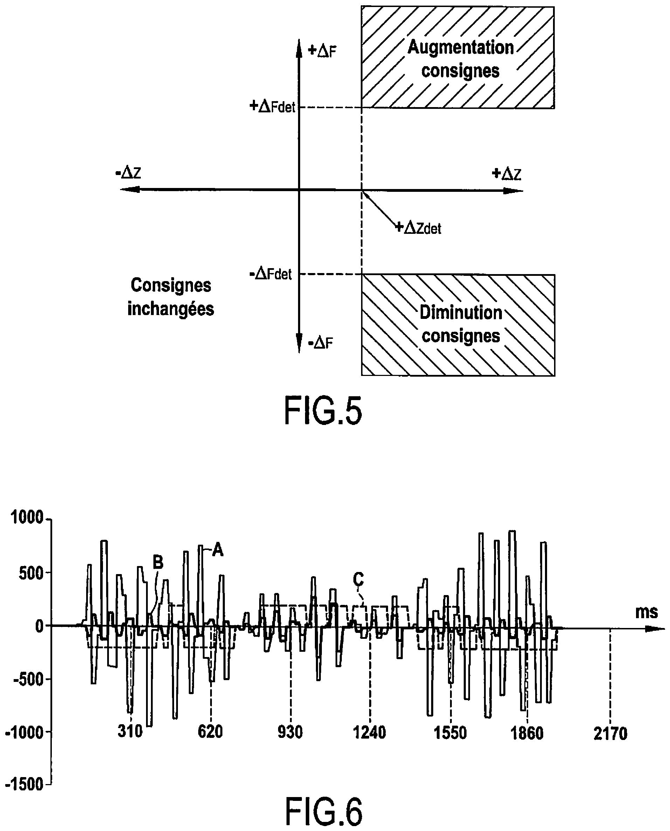

La figure 5 est un graphique montrant les conditions du contrôle de l'amplitude des signaux de consigne en courant et en tension en fonction de la variation de l'impédance Δ_{Z} et de la fréquence Δ_{F} du signal ultrasonore dans le transducteur comme décrit ci-avant.

Le contrôle d'amplitude des signaux de consigne est applicable à des signaux ultrasonores d'amplitude constante et des signaux ultrasonores modulés. Dans ce dernier cas, la fréquence de modulation est de préférence supérieure ou égale à une valeur déterminée afin de permettre la détermination d'une valeur moyenne de mesure de l'impédance et la fréquence du signal dans le transducteur. Par exemple, lorsque le module 320 détermine une valeur moyenne de mesure sur une période de 100 millisecondes, la fréquence de modulation du signal ultrasonore est égale ou supérieure à 25 Hz.

A titre d'exemple non limitatif, après expérimentation, la valeur prédéterminée de variation d'impédance Δ_{Zdet}, peut être fixée à 26 ohms tandis que la valeur prédéterminée de variation de fréquence Δ_{Fdet} peut être fixée à 12 Hz, et ce pour une détermination d'une valeur moyenne de mesure de l'impédance et la fréquence du signal dans le transducteur toutes les 100 ms. Toujours à titre d'exemple non limitatif, l'augmentation ou la diminution de l'amplitude des signaux de consigne peut être fixée à 20% de la valeur de consigne initiale, c'est-à-dire celle paramétrée dans l'appareil de traitement à ultrason en fonction du type de traitement sélectionné. Les valeurs indiquées ci-avant ont été déterminées pour un appareil de traitement à ultrasons destiné à réaliser des traitements dentaires. Ces valeurs peuvent bien entendu être différentes dans le cas d'autres types de traitements chirurgicaux comme pour des chirurgies maxillo-faciales ou des chirurgies orthopédiques.

La figure 6 illustre un exemple de mise en œuvre du contrôle d'amplitude des signaux de consigne de l'invention dans un appareil de traitement dentaire à ultrasons, ici l'appareil « Piezotome Solo » commercialisé par la société SATELEC^{®} et destiné à la chirurgie pré-implantaire. Sur figure 6, la courbe A représente la variation d'impédance Δ_{Z} mesurée dans le transducteur, la courbe B représente la variation de fréquence Δ_{F} mesurée dans le transducteur et la courbe C représente la commande appliquée sur l'amplitude des signaux de consigne en courant et en tension délivrés au transducteur. Les augmentations de l'amplitude des signaux de consigne correspondent aux parties de la courbe C qui sont au-dessus de l'axe des abscisses. Les diminutions de l'amplitude des signaux de consigne correspondent aux parties de la courbe C qui sont en dessous de l'axe des abscisses. Les parties de la courbe C confondues avec l'axe des abscisses correspondent aux instants où l'amplitude des signaux de consigne n'est pas modifiée.

Le module de contrôle d'amplitude des signaux de consigne 320 peut être désactivé lors du paramétrage de l'appareil de traitement à ultrasons.

## Revendications

1. Appareil de traitement à ultrasons (100) comprenant au moins une pièce à main chirurgicale (200), un insert ultrasonique (130) et un générateur d'ultrasons (110), la pièce à main comprenant un transducteur piézoélectrique (220) constitué d'une partie distale (232) sur laquelle est fixé l'insert ultrasonique (130) et d'un moteur piézoélectrique (222) relié au générateur d'ultrasons (110) et couplé mécaniquement à la partie distale (232), ledit moteur piézoélectrique transmettant à l'insert des ondes ultrasonores définies en fonction de signaux de consigne de courant et de tension (Sciu) délivrés par le générateur d'ultrasons (110) au moteur piézoélectrique (222), ledit appareil de traitement à ultrasons comprenant en outre un module de contrôle d'amplitude des signaux de consigne (320) configuré pour :
- calculer une variation de la fréquence (Δ_{F}) et de l'impédance (Δz) d'un signal ultrasonore dans le transducteur piézoélectrique,
- comparer la variation de l'impédance du signal ultrasonore à une valeur prédéterminée de variation d'impédance (Δ_{Zdet}),
- comparer la variation de la fréquence du signal ultrasonore à une valeur prédéterminée de variation de fréquence (Δ_{Fdet}),
- augmenter l'amplitude des signaux de consigne de courant et de tension (Sciu) délivrés par le générateur d'ultrasons (110) lorsque la variation de l'impédance (Δz) du signal ultrasonore calculée est supérieure à la valeur prédéterminée de variation d'impédance (Δ_{Zdet}) et lorsque la variation de la fréquence (Δ_{F}) du signal ultrasonore calculée est supérieure à la valeur prédéterminée de variation de fréquence (Δ_{Fdet}), et
- diminuer l'amplitude des signaux de consigne de courant et de tension (Sciu) délivrés par le générateur d'ultrasons lorsque la variation de l'impédance (Δz) du signal ultrasonore calculée est supérieure à la valeur prédéterminée de variation d'impédance (Δ_{Zdet}) et lorsque la variation de la fréquence (Δ_{F}) du signal ultrasonore calculée est inférieure à la valeur prédéterminée de variation de fréquence (Δ_{Fdet}).

2. Appareil selon la revendication 1, dans lequel les valeurs moyennes de la fréquence et de l'impédance du signal ultrasonore dans le transducteur piézoélectrique (220) sont mesurées toutes les 100 millisecondes.

3. Appareil selon la revendication 2, dans lequel la valeur prédéterminée de variation de fréquence (Δ_{Fdet}) est égale à 12 hertz.

4. Appareil selon la revendication 2 ou 3, dans lequel la valeur prédéterminée de variation d'impédance (Δ_{Zdet}) est égale à 26 ohms.

5. Appareil selon l'une quelconque des revendications 1 à 4, dans lequel l'amplitude des signaux de consigne de courant et de tension (Sciu) délivrés par le générateur d'ultrasons (110) est augmentée de 20% lorsque la variation de la fréquence (Δ_{F}) du signal ultrasonore calculée est supérieure à la valeur prédéterminée de variation de fréquence (Δ_{Fdet}) et lorsque la variation de l'impédance (Δz) du signal ultrasonore calculée est supérieure à la valeur prédéterminée de variation d'impédance (Δ_{Zdet}), et dans lequel l'amplitude des signaux de consigne de courant et de tension (Sciu) délivrés par le générateur d'ultrasons de la consigne est diminuée de 20% lorsque la variation de la fréquence (Δ_{F}) du signal ultrasonore mesurée est inférieure à la valeur prédéterminée de variation de fréquence (Δ_{Fdet}) et lorsque la variation de l'impédance (Δz) du signal ultrasonore mesurée est supérieure à la valeur prédéterminée de variation d'impédance (Δ_{Zdet}).

## Patentansprüche

1. Vorrichtung (100) zur Ultraschallbehandlung, umfassend zumindest ein chirurgisches Handstück (200), einen Ultraschalleinsatz (130) und einen Ultraschallgenerator (110), wobei das Handstück einen piezoelektrischen Wandler (220), der aus einem distalen Teil (232), auf welchem der Ultraschalleinsatz (130) befestigt ist, und einem piezoelektrischen Motor (222) besteht, der mit dem Ultraschallgenerator (110) verbunden und mechanisch mit dem distalen Teil (232) gekoppelt ist, wobei der piezoelektrische Motor auf den Einsatz Ultraschallwellen überträgt, die in Abhängigkeit von Strom- und Spannungs-Stellsignalen (S_{CIU}) definiert werden, die von dem Ultraschallgenerator (110) an den piezoelektrischen Motor (222) geliefert werden,
wobei die Vorrichtung zur Ultraschallbehandlung ferner ein Modul (320) zur Steuerung der Amplitude der Stellsignale umfasst, das dazu ausgestaltet ist:
- eine Variation der Frequenz (Δ_{F}) und der Impedanz (Δ_{Z}) eines Ultraschallsignals in dem piezoelektrischen Wandler zu berechnen,
- die Variation der Impedanz des Ultraschallsignals mit einem vorbestimmten Wert der Variation der Impedanz (Δ_{Zdet}) zu vergleichen,
- die Variation der Frequenz des Ultraschallsignals mit einem vorbestimmten Wert der Variation der Frequenz (Δ_{Fdet}) zu vergleichen,
- die Amplitude der Strom- und Spannungs-Stellsignale (S_{CIU}), die von dem Ultraschallgenerator (110) geliefert werden, zu erhöhen, wenn die berechnete Variation der Impedanz (Δ_{Z}) des Ultraschallsignals höher als der vorbestimmte Wert der Variation der Impedanz (Δ_{Zdet}) ist und wenn die berechnete Variation der Frequenz (Δ_{F}) des Ultraschallsignals höher als der vorbestimmte Wert der Variation der Frequenz (Δ_{Fdet}) ist, und
- die Amplitude der Strom- und Spannungs-Stellsignale (S_{ClU}), die von dem Ultraschallgenerator geliefert werden, zu verringern, wenn die berechnete Variation der Impedanz (Δ_{Z}) des Ultraschallsignals höher als der vorbestimmte Wert der Variation der Impedanz (Δ_{Zdet}) ist und wenn die berechnete Variation der Frequenz (Δ_{F}) des Ultraschallsignals niedriger als der vorbestimmte Wert der Variation der Frequenz (Δ_{Fdet}) ist.

2. Vorrichtung nach Anspruch 1, wobei die mittleren Werte der Frequenz und der Impedanz des Ultraschallsignals in dem piezoelektrischen Wandler (220) alle 100 Millisekunden gemessen werden.

3. Vorrichtung nach Anspruch 2, wobei der vorbestimmte Wert der Variation der Frequenz (Δ_{Fdet}) gleich 12 Hertz ist.

4. Vorrichtung nach Anspruch 2 oder 3, wobei der vorbestimmte Wert der Variation der Impedanz (Δ_{Zdet}) gleich 26 Ohm ist.

5. Vorrichtung nach einem der Ansprüche 1 bis 4, wobei die Amplitude der Strom- und Spannungs-Stellsignale (S_{ClU}), die von dem Ultraschallgenerator (110) geliefert werden, um 20 % erhöht wird, wenn die berechnete Variation der Frequenz (Δ_{F}) des Ultraschallsignals höher als der vorbestimmte Wert der Variation der Frequenz (Δ_{Fdet}) ist und wenn die berechnete Variation der Impedanz (Δ_{Z}) des Ultraschallsignals höher als der vorbestimmte Wert der Variation der Impedanz (Δ_{Zdet}) ist, und wobei die Amplitude der Strom- und Spannungs-Stellsignale (S_{ClU}), die von dem Stell-Ultraschallgenerator geliefert werden, um 20 % verringert wird, wenn die Variation der Frequenz (Δ_{F}) des gemessenen Ultraschallsignals niedriger als der vorbestimmte Wert der Variation der Frequenz (Δ_{Fdet}) ist und wenn die Variation der Impedanz (Δ_{Z}) des gemessenen Ultraschallsignals höher als der vorbestimmte Wert der Variation der Impedanz (Δ_{Zdet}) ist.

## Claims

1. An ultrasound treatment appliance (100) comprising at least a surgical handpiece (200), an ultrasound insert (130), and an ultrasound generator (110), the handpiece including a piezoelectric transducer (220) constituted by a distal portion (232) having the ultrasound insert (130) fastened thereto and a piezoelectric motor (222) connected to the ultrasound generator (110) and mechanically coupled to the distal portion (232), said piezoelectric motor transmitting ultrasound waves to the insert, which waves are defined as a function of current and voltage setpoint signals (S_{CIU}) delivered by the ultrasound generator (110) to the piezoelectric motor (222),
said ultrasound treatment appliance further comprising a module for controlling the amplitudes of the setpoint signals (320) and configured to:
- calculate a variation in the frequency (Δ_{F}) and the impedance (Δz) of the control signal in the piezoelectric transducer;
- compare the impedance variation of the ultrasound signal with a predetermined impedance variation value (Δ_{Zdet}) ;
- compare the variation in the frequency of the ultrasound signal with a predetermined frequency variation value (Δ_{Fdet});
- increase the amplitudes of the current and voltage setpoint signals (S_{CIU}) delivered by the ultrasound generator (110) when the calculated variation in the impedance (Δz) of the ultrasound signal is greater than the predetermined impedance variation value (Δ_{Zdet}) and when the calculated variation in the frequency (Δ_{F}) of the ultrasound signal is greater than the predetermined frequency variation value (Δ_{Fdet}); and
- decrease the amplitudes of the current and voltage setpoint signals (S_{CIU}) delivered by the ultrasound generator when the calculated variation in the impedance (Δz) of the ultrasound signal is greater than the predetermined impedance variation value (Δ_{Zdet}) and when the calculated variation in the frequency (Δ_{F}) of the ultrasound signal is less than the predetermined frequency variation value (Δ_{Fdet}).

2. An appliance according to claim 1, wherein the mean values of the frequency and the impedance of the ultrasound signal in the piezoelectric transducer (220) are measured once every 100 ms.

3. An appliance according to claim 2, wherein the predetermined frequency variation value (Δ_{Fdet}) is equal to 12 Hz.

4. An appliance according to claim 2 or claim 3, wherein the predetermined impedance variation value (Δ_{Zdet}) is equal to 26 Ω.

5. An appliance according to any one of claims 1 to 4, wherein the amplitudes of the current and voltage setpoint signals (S_{CIU}) delivered by the ultrasound generator (110) are increased by 20% when the calculated variation in the frequency (Δ_{F}) of the ultrasound signal is greater than the predetermined frequency variation value (Δ_{Fdet}) and when the calculated variation in the impedance (Δz) of the ultrasound signal is greater than the predetermined impedance variation value (Δ_{Zdet}), and wherein the amplitudes of the current and voltage setpoint signals (S_{CIU}) delivered by the ultrasound setpoint generator are decreased by 20% when the measured variation in the frequency (Δ_{F}) of the ultrasound signal is less than the predetermined frequency variation value (Δ_{Fdet}) and when the measured variation in the impedance (Δz) of the ultrasound signal is greater than the predetermined impedance variation value (Δ_{Zdet}).
